# EUROPEAN PATENT APPLICATION

(11) **EP 1 290 978 A2**
(43) Date of publication of application: **12.03.2003**
(21) Application number: 02020026.7
(22) Date of filing: 06.09.2002
(51) Int. Cl.: A61B 10/00

(54) **Needle set for biopsy**

(30) Priority: 07.09.2001 IT BO20010531
(71) Applicant: Gallini S.r.l., 46100 Mantova (IT)
(72) Inventor: Avaltroni, Paolo, 46100 Mantova (IT)
(74) Representative: Dall'Olio, Giancarlo

(57) **Abstract**

A needle set for biopsy includes a hollow cannula (3) and a plunger (7), aimed at being introduced slidingly into the cannula (3). The plunger (7) has, at its distal portion, a lateral cavity (9) aimed at receiving a biopsy sample to be taken. The cannula (3) and plunger (7) are also provided, at their respective proximal ends (3b,7b), with relative heads, a cannula head (4) and a plunger head (8), respectively. The needle set (100) includes also a shaft (10), which extends for a predetermined length from the plunger head (8), parallel to the plunger (7) and toward the distal portion thereof. The shaft (10) is slidingly engaged in a longitudinal hole (5) made in the cannula head (4) parallel to the cannula (3), so as to guide and limit the stroke of the plunger (7) inside the cannula.

## Description

The present invention relates to the technical field concerning the production of needle-equipped instruments for biopsy operations, i.e. for removing, from a patient's body, samples of biological tissue to be analysed.

In particular, the invention relates to a needle, which can be used in a special type of such instruments, consisting of an outer cannula and an inner plunger.

Many needle instruments for removing bioptical samples have heretofore been provided.

The biopsy needles usually include a cannula, hollow inside, which is introduced into the patient's body, in a region corresponding to an organ or a new formation, from which a sample is to be taken.

The sample is held within the cannula by means of techniques depending on the type of the instrument, then the cannula is removed together with the sample to be analysed.

The introduction of the cannula is often facilitated by providing a plunger, which slides inside the cannula and has a distal portion protruding from the cannula.

Moreover, this allows reaching the sample area without taking tissues different from the one to be taken.

A particularly used type of instrument, specially fit for sampling soft tissues, is called a "guillotine".

This instrument includes a lateral cavity, situated near the distal end of the plunger.

While the needle is introduced into the patient's body, the cavity remains completely within the cannula.

When the tip of the cannula reaches the sample area, the plunger is moved forward thus uncovering the cavity, which is substantially filled with the tissue to be sampled.

At this point, the cannula is moved quickly forward, by means of various known mechanisms, and cuts the portion of tissue contained in the cavity of the plunger like a guillotine, separating it from the remaining tissue.

Then, the cannula and the plunger are withdrawn together and keep the cut bioptic sample within them.

In order to perform the above described operation in a safe and recurring way and thus to avoid errors which could cause the loss of sample or taking an insufficient quantity thereof, there are devices which perform the final part of the operation in an automatic way.

Basically, the needle is introduced manually by an expert operator, under radiographic or ultrasonic echography control, maintaining the plunger in the introduction position.

When the distal portion of the cannula and the plunger are located in the region of the tissue to sample, the needle is introduced into the automatic device.

The automatic device features two sliding elements, situated thereinside and suitably spaced out.

One sliding element is aimed at receiving the head of the plunger and the other is aimed at receiving the head of the cannula.

The needle is mounted by means of suitable male-female couplings, made in the sliding elements and on the above mentioned plunger and cannula heads.

The operator holds the device in his hand, in a substantially stationary position.

At the suitable moment, the operator starts the automatic execution of the plunger forwarding and subsequent cannula quick forwarding, so as to obtain the bioptic sample, which is fitted between the cavity of the plunger and the inner wall of the cannula.

Contrary to the automatic device, the plunger and the cannula are disposable, and are usually introduced in the same package, but they can be released and detached one from the other.

Because the plunger and the cannula slide freely one inside the other, it is difficult for the operator to maintain them in the correct position while they are introduced into the patient's body during the execution of the above first manual step of the sampling.

Moreover, once the needle has been positioned in the sampling area, the mutual distance between the plunger and cannula heads must be exactly the same as the distance between the positions of the relative sliding members of the automatic device.

Obviously, what above is difficult to obtain, if the plunger is free to slide inside the cannula.

In order to avoid, or at least minimize, the above mentioned drawbacks, the needle is usually supplied with its own spacer, which acts also as a handle during the needle manipulation.

The spacer is equipped with gripping, pliers like means, suitably spaced out.

Each of the gripping means can be coupled with corresponding notches made in the plunger and cannula heads.

The spacers of the above described type allow to identify precisely the mutual position of the couple plunger-cannula, however they still feature some drawbacks.

Actually, they do not have a good resistance to axial thrust and can detach during the needle positioning operations, thus making the plunger lose its correct position with respect to the cannula.

The object of the present invention is to propose a compound needle for execution of bioptic samples, which allows to avoid the above mentioned drawbacks.

Another object of the present invention is to propose a needle which is simple to use and cheap to produce.

The above mentioned objects are wholly obtained, in accordance with the contents of the claim 1, by a compound needle for bioptic sampling, which includes a hollow cannula and a plunger, introduced slidingly into the cannula, said cannula and plunger being also provided, at the respective proximal ends, with related heads, said compound needle including guiding means, which extend for a predetermined length from the plunger head, parallel to the plunger and toward the distal portion thereof, said guiding means being also engaged slidingly in a longitudinal seat made in the cannula head parallel to the cannula, so as to guide and limit the stroke of the plunger inside the cannula.

The characteristic features of the invention, resulting from claims, are pointed out in the following detailed description with reference to the enclosed drawings, in which:
- Figure 1 is a view of a compound needle obtained according to the present invention, in an introduction position;
- Figure 2 is a view of the needle of Figure 1 with the distal portion of the plunger in an extraction position;
- Figure 3 is a view of a compound needle according to another embodiment.

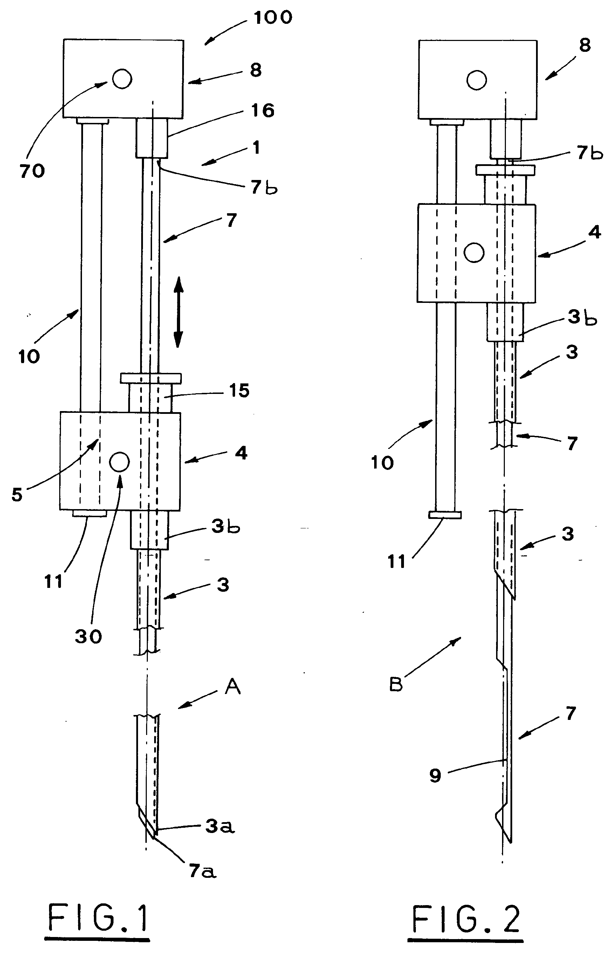

With reference to Figures 1 and 2, the reference numeral 100 indicates herein a needle for taking bioptic samples according to a first embodiment of the invention, including a cannula 3 and a plunger 7.

The cannula 3 is internally hollow and the plunger 7 is slidably inserted therein, moving between a rear position A (Figure 1) and a fore position B (Figure 2).

The biopsy needle 100 is especially aimed at being used with known automatic snap-operated devices for taking samples, whose functionality are widely known and consequently, they will not be described in detail.

With reference thereto, the rear position A defines the correct position for the introduction of the needle 100 in the automatic device, when the latter is in its position ready to be snap-operated.

The dimensions of the plunger 7 allow the sharp end 7a thereof to protrude slightly from the distal end 3a of the cannula 3, when the plunger 7 is in the above rear position A, while its proximal end 7b is set at a distance from the proximal end 3b of the cannula 3.

When the plunger 7 is in the fore position B, the proximal ends 3b, 7b are in close position, while a distal portion of the plunger 7 protrudes from the above distal end 3a. The distal portion of the plunger 7 features, made therein, a lateral cavity 9, aimed at receiving the bioptic sample to be taken.

The cannula 3 and the plunger 7 are also equipped, at their proximal ends 3b, 7b, with a related head 4, for the cannula, and a related head 8 for the plunger, respectively.

The heads are equipped with coupling means 30, 70, which allow their coupling with the corresponding device for automatic sampling.

According to the embodiment shown in the figures, the coupling means 30, 70 include substantially an equal number of holes having predetermined diameter, aimed at engaging with corresponding pins made in the automatic device.

However, it is to be noted that, in relation to the particular device, to which the present compound needle is to be joined, the coupling means 30, 70 can include characteristic conformations of the cannula head 4 and the plunger head 8, or other known coupling configurations.

Guiding means 10 are fastened to the plunger head 8 and include a shaft having a substantially cylindrical section, which extends toward the sharp end 7a of the plunger 7, parallel thereto and at a predetermined distance therefrom.

The shaft 10 is also engaged slidably with a longitudinal seat 5, which includes a through hole made in the cannula head 4 parallel to the cannula 3, to guide and limit the stroke of the plunger 7 inside the cannula 3.

The length of the shaft 10 allows the plunger 3 to perform the whole stroke between the above mentioned rear position A and fore position B.

Moreover, at its distal end, the shaft 10 has an enlargement 11 whose section is bigger than the section of the above mentioned longitudinal hole 5.

The enlargement 11 is aimed at preventing the shaft 10 from going out from the above mentioned longitudinal hole 5 and defines precisely the rear position A of the plunger 3, which, as it has been already said, corresponds to the correct position of introduction of the needle 100 into the automatic sampling device.

The cannula head 4 features, on the side opposite to the one from which the cannula 3 protrudes as its prolongation, a substantially cylindrical and hollow inside seat 15.

The seat 15 is aimed at receiving slidably the plunger 7 and at receiving a corresponding protrusion 16, situated between the proximal end 7b of the plunger 7 and the plunger head 8, so as to act as abutment between the latter and the cannula head 4.

The use of the compound needle 100 obtained in the above described way is easy to understand.

The operator, who is to take a bioptic sample, brings the compound needle to the rear position A (Figure 1), by withdrawing the plunger 7 from the cannula 3 until the cannula head 8 goes in abutment against the enlargement 11 of the shaft 10.

The operator can easily maintain the above position by gripping the needle 100 between the thumb and forefinger in the region corresponding to the cannula head 8, taking care to grip also the shaft 10 and the part of the plunger 7 parallel thereto.

In this way, the operator can introduce the needle, with its components in the correct position, into the patient's body until the operation position is reached.

At this point, the operator introduces the needle 100 into the snap-operated sampling device and he/she can start the bioptic sample taking operation.

Therefore, the sampling device can perform the operation with known operation sequence, which usually includes a first step, during which the plunger 7 forwards up to the fore position B (Figure 2) and subsequently the cannula 3 moves quickly forward, until the needle is brought back to the position A.

The last step allows separation of a portion of tissue contained in the longitudinal cavity 9 of the plunger 7, and then to take it out of the patient's body for subsequent analysis.

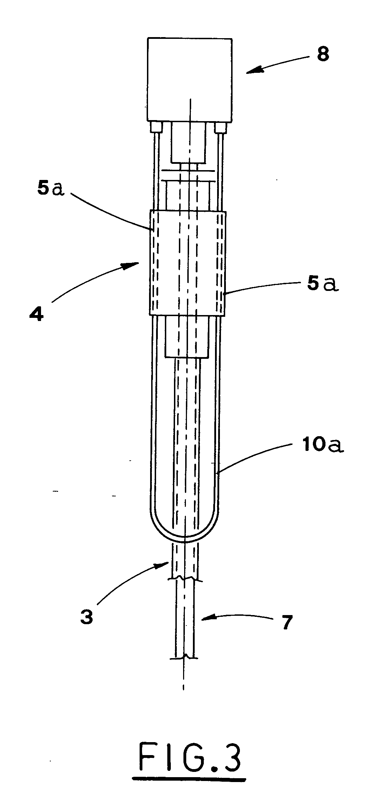

Figure 3 shows a second embodiment of the compound needle 100 according to the invention.

According to this embodiment, guiding means 10a include a rigid thread, preferably metallic, folded like "U" and extending longitudinally and parallel to the cannula 3 and the needle 7.

The ends of the rigid thread 10a are fastened to the plunger head 8.

There are also guiding means 5a which include a pair of longitudinal grooves, made laterally in the cannula head 4 and aimed at receiving slidably the opposite runs of the above mentioned rigid thread 10a.

The above described compound needle 100 can be correctly positioned during the manual step of the bioptic sampling, in a simple and cheap way, and with use of not cumbersome elements, allowing the operator to grip safely the group cannula-plunger during this step.

Moreover, the position of introduction of the group cannula-plunger into the relative device for automatic sampling is defined precisely.

## Claims

1. Compound needle for bioptic sampling, the needle including a hollow cannula (3) and a plunger (7), aimed at being introduced slidingly into said cannula (3), and provided, at its distal portion, with a lateral cavity (9) aimed at receiving a bioptic sample to be taken, said cannula (3) and plunger (7) being also provided, at their respective proximal ends (3b,7b), with relative heads, a cannula head (4) and a plunger head (8), respectively, said compound needle (100) being **characterized in that** it includes guiding means (10,10), which extend for a predetermined length from the plunger head (8), parallel to said plunger (7) and toward said distal portion thereof, said guiding means (10a) also slidingly engaging at least one longitudinal seat (5,5a) made in said cannula head (4) parallel to said cannula (3), so as to guide and limit the stroke of said plunger (7) inside the cannula.

2. Compound needle, according to claim 1, **characterized in that** said guiding means (10) include a shaft having a predetermined length and **in that** said longitudinal seat (5) includes a through hole made in said cannula head (4).

3. Compound needle, according to claim 2, **characterized in that** said shaft (10) and through hole (5) have circular section.

4. Compound needle, according to claim 1, **characterized in that** said guiding means (10a) include a "U"-like rigid thread with ends fastened to said plunger head (8), and **in that** said longitudinal seat (5a) includes a pair of grooves made laterally in said cannula head (4) and aimed at receiving slidably the opposite runs of said rigid thread (10a).

5. Compound needle, according to claims 2 or 4, **characterized in that** said shaft (10) includes, at its distal end, locking means (11) aimed at preventing the cannula head (4) from going out from said shaft (10).

6. Compound needle, according to claim 5, **characterized in that** said locking means (11) include added material forming an enlargement having section bigger than the section of said longitudinal hole (5).
